# EUROPEAN PATENT APPLICATION

(11) **EP 4 825 965 A1**
(43) Date of publication of application: **21.10.2026**
(21) Application number: 24903608.8
(22) Date of filing: 05.12.2024
(51) Int. Cl.: C12N 15/11, C12N 1/21, C12N 15/63, C12P 19/34, C12P 21/02

(54) **MODIFIED 5'-UNTRANSLATED REGION AND METHOD FOR PRODUCING TARGET SUBSTANCE USING SAME**

(30) Priority: 15.12.2023 JP 2023212488
(71) Applicant: Kao Corporation, Tokyo 103-8210 (JP)
(72) Inventor: HONJO, Hiroshi, Tokyo 103-8210 (JP); KAWAHARA, Akihito, Tokyo 103-8210 (JP)
(74) Representative: Vossius & Partner Patentanwälte Rechtsanwälte mbB
(86) International application number: PCT/JP2024/043094
(87) International publication number: WO 2025/126951

(57) **Abstract**

A DNA molecule comprising a modified promoter, in which the modified promoter comprises a modified 5'-UTR containing a heterologous polynucleotide, and the heterologous polynucleotide contains the nucleotide sequence of SEQ ID NO: 70, or a nucleotide sequence having at least 80% identity with the nucleotide sequence of SEQ ID NO: 70.

## Description

### Field of the Invention

The present invention relates to a DNA molecule containing a modified 5'-untranslated region or a promoter containing the modified 5'-untranslated region, and a method for producing a target substance using the same.

### Background of the Invention

In industrial production of substances by microorganisms, improvement of productivity is an important issue. Many studies have been conducted on modification of an expression control region such as a promoter for improving the expression of a target gene.

Patent Literature 1 indicates that the expression of a target gene is improved by a modified promoter obtained by modifying a catabolite responsive element (cre)-like sequence derived from an alkaline cellulase gene of *Bacillus* sp. KSM-S237 strain (FERM BP-7875) and KSM-64 strain (FERM BP-2886). Patent Literature 2 indicates that the expression of a target gene is improved by a modified promoter obtained by inserting a base(s) between positions 326 and 330 of a nucleotide sequence of an alkaline cellulase gene promoter region of KSM-64 strain. Patent literatures 3 and 4 indicate that a 5'-untranslated region (5'-UTR) sequence obtained from a *Bacillus subtilis* aprE gene is operably linked to a heterologous gene to improve the expression of the gene. Patent Literature 5 indicates that a modified mRNA processing/stabilizing sequence to which a Shine-Dalgarno sequence is added is connected downstream of a promoter region of a target gene and upstream of a ribosome binding region to improve the expression of the target gene. Patent Literature 6 discloses a method in which a specific DNA sequence which forms a stem-loop is introduced 7 or more nucleotides downstream of a transcription initiation site of a gene related to synthesis of a target substance, whereby the production of the target substance is increased. Non Patent Literature 1 indicates that in *Bacillus subtilis,* introduction of a Shine-Dalgarno-like sequence into the 5'-UTR of sacR, repressor gene of sacB, controlled the expression of sacB, and the expression was increased or decreased depending on the number of the sequences introduced and the introduction position. Non Patent Literature 2 indicates that in *Bacillus* bacteria, the expression of a target gene is improved by introduction of a plurality of sequences containing a ribosome binding site (RBS) and a start codon in a 5'-UTR region of the target gene.

(Patent Literature 1) JP-A-2011-103875
(Patent Literature 2) JP-A-2000-078981
(Patent Literature 3) JP-A-2018-505686
(Patent Literature 4) JP-A-2020-534821
(Patent Literature 5) WO 2008/140615
(Patent Literature 6) JP-A-2009-518019
(Non Patent Literature 1) Lett Appl Microbiol, 2005, 41(2): 221-226
(Non Patent Literature 2) Nucleic Acids Research, 2022, 50(20): 11979-11990

### Summary of the Invention

In an embodiment, the present invention provides a DNA molecule comprising a modified promoter,
the modified promoter comprising a modified 5'-UTR containing a heterologous polynucleotide,
the heterologous polynucleotide comprising a nucleotide sequence of SEQ ID NO: 70, or a nucleotide sequence having at least 80% identity with the nucleotide sequence of SEQ ID NO: 70.

In another embodiment, the present invention provides a transformant comprising the DNA molecule.

In still another embodiment, the present invention provides a method for producing a target substance, comprising culturing the transformant.

In still another embodiment, the present invention provides a method for producing a modified promoter,
the method comprising modifying a 5'-UTR contained in a parent promoter,
wherein the modifying a 5'-UTR comprises substituting a part or a whole of the 5'-UTR with a heterologous polynucleotide, or adding the heterologous polynucleotide to the 5'-UTR,
the heterologous polynucleotide comprising a nucleotide sequence of SEQ ID NO: 70, or a nucleotide sequence having at least 80% identity with the nucleotide sequence of SEQ ID NO: 70,
the parent promoter comprising a 5'-UTR which does not contain the heterologous polynucleotide.

### Brief Description of Drawings

Fig. 1 illustrates a P_{SP64} promoter containing a modified 5'-UTR constructed in Example 2. The shaded area represents a region substituted for a parent sequence.
Fig. 2 illustrates a spoVG promoter containing a modified 5'-UTR constructed in Example 3. The shaded area represents a region substituted for a parent sequence.
Fig. 3 illustrates a P_{SP64} promoter containing a modified 5'-UTR constructed in Example 7, and heterologous RBS. The shaded area represents a region substituted for a parent sequence, and the black bar represents heterologous RBS.
Fig. 4 illustrates a method for producing a prsA overexpression strain.
Fig. 5 illustrates improvement of KP43 protease productivity by a P_{SP64} promoter containing a modified 5'-UTR.
Fig. 6 illustrates improvement of KP43 protease productivity by a spoVG promoter containing a modified 5'-UTR.
Fig. 7 illustrates improvement of amylase productivity by a P_{SP64} promoter containing a modified 5'-UTR.
Fig. 8 illustrates improvement of amylase productivity by a spoVG promoter containing a modified 5'-UTR.
Fig. 9 illustrates improvement of Cry5B productivity by a P_{SP64} promoter containing a modified 5'-UTR.
Fig. 10 illustrates improvement of lipase productivity by a P_{SP64} promoter containing a modified 5'-UTR.
Fig. 11 illustrates improvement of Cry5B productivity by a P_{SP64} promoter containing a modified 5'-UTR and additional RBS.

### Detailed Description of the Invention

All patent documents, non-patent documents and other publications cited herein are incorporated herein by reference in their entirety.

As used herein, the identity between amino acid sequences or nucleotide sequences is calculated by a Lipman-Pearson method (Science, 1985, 227: 1435-1441). Specifically, the identity is calculated by analysis using the search homology program of gene information processing software GENETYX Ver. 12 (NIHON SERVER CORPORATION) by setting the unit size to compare (ktup) to 2.

As used herein, the term "at least 80% identity" pertaining to an amino acid sequence and a nucleotide sequence refers to an identity of 80% or more, preferably 85% or more, more preferably 90% or more, still more preferably 95% or more, still more preferably 98% or more, and still more preferably 99% or more.

As used herein, unless otherwise specified, the term "one or several" used in relation to deletion, substitution, addition or insertion of an amino acid residue or a nucleotide in an amino acid sequence or a nucleotide sequence may mean being preferably from 1 to 5, more preferably from 1 to 4, still more preferably from 1 to 3, and still more preferably 1 or 2. As used herein, the "addition" of an amino acid residue or a nucleotide includes the addition of an amino acid residue or a nucleotide to one terminus and both termini of a sequence.

As used herein, the "corresponding position" or the "corresponding region" on an amino acid sequence or a nucleotide sequence can be determined by aligning a target sequence and a reference sequence (for example, the amino acid sequence of SEQ ID NO: 3) so as to give the maximum homology. The alignment of an amino acid sequence or a nucleotide sequence can be carried out using a known algorithm and a procedure for the alignment is known to those skilled in the art. For example, the alignment can be performed by using a Clustal W multiple alignment program (Thompson, J. D. et al, 1994, Nucleic Acids Res. 22: 4673-4680) in a default setting. Clustal W can be used, for example, on the Web site of the DNA Data Bank of Japan (DDBJ [www.ddbj.nig.ac.jp/searches-j.html]) operated by National Institute of Genetics. The position of the target sequence aligned with an arbitrary position of the reference sequence through the alignment described above is regarded as the "position corresponding to" the arbitrary position. A region sandwiched between corresponding positions, or a region consisting of a corresponding motif is regarded as a corresponding region.

As used herein, the term "amino acid residue" means 20 proteinogenic amino acid residues, which are alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophane (Trp or W), tyrosine (Tyr or Y), and valine (Val or V).

As used herein, the "promoter" is a DNA sequence having a function of controlling the expression of a gene (a polynucleotide encoding an expression product). In general, the "promoter" of a certain gene exists upstream of an open reading frame (ORF) of the gene, and controls the expression of the ORF. The promoter may contain regions such as a transcription start site and a 5'-untranslated region (5'-UTR), or may be defined as a region further including an enhancer, a cis-element and the like. The enhancer or the cis-element are located upstream of the transcription start site, and a transcription factor or the like binds to the enhancer or the cis-element to improve the activity of the promoter. The activation of the promoter promotes transcription of a gene to mRNA. The 5'-UTR is a region downstream of the transcription start site and just before the start codon. The 5'-UTR itself is transcribed to mRNA, but is not translated, and functions as a regulatory region when mRNA is translated into a protein. The 5'-UTR in *Bacillus* bacteria contains a Shine-Dalgarno (SD) sequence which is a ribosome binding site.

As used herein, the term "open reading frame (ORF)" refers to a region from a start codon to a stop codon. ORF contains a coding region (coding sequence (CDS)) of a gene.

As used herein, the term "expression control region" (or simply "control region") refers to a DNA sequence having a function of controlling transcription or translation of a gene, and includes a promoter, 5'-UTR constituting the promoter, a transcription start site, a ribosome binding site and other sites and regions, an enhancer and a cis-element which improve the transcriptional activity of the promoter, and a 3'-untranslated region (3'-UTR).

As used herein, the terms "upstream" and "downstream" used in relation to a gene, or control regions such as a promoter and 5'-UTR refer to the 5'-side and the 3'-side, respectively, of the gene or the region. Unless otherwise specified, the upstream and the downstream of a gene are not limited to upstream and downstream regions adjacent to ORF of the gene. Unless otherwise specified, the upstream and the downstream pertaining to a promoter and 5'-UTR are not limited to an upstream region adjacent to the 5'-end of the region and a downstream region adjacent to the 3'-end of the region, respectively.

As used herein, the phrase that a control region and a gene are "operably linked" means that a gene (ORF) and a control region are linked such that the gene can be expressed under the control of the control region. As used herein, the phrase that individual regions contained in a control region (for example, a promoter and 5'-UTR) are "operably linked" means that in the control region, the individual regions are linked such that expression of a gene (ORF) downstream of the regions can be controlled. Therefore, basically, the term "gene" operably linked to the control region, as used herein, refers to ORF. A procedure for "operably linking" a gene and a control region or the regions in the control region is well known to those skilled in the art.

As used herein, the term "expression cassette" refers to a polynucleotide construct for controlling the expression of a target gene contained in the expression cassette. Normally, the expression cassette contains a target gene to be expressed, and a control region for controlling the expression of the gene. The control region preferably contains a promoter, and the promoter is located upstream of ORF of the target gene, and is operably connected to the ORF. For example, in the case where the target gene encodes a polynucleotide encoding a preproprotein containing a signal peptide, the promoter is located upstream of a region encoding the signal peptide, is operably connected to a polynucleotide encoding the preproprotein, and controls the expression of the preproprotein. The expression cassette may contain a 3'-untranslated region (3'-UTR) of the target gene. Preferably, the expression cassette has a restriction enzyme recognition site at one end for allowing the expression cassette to be inserted into a vector or genomic DNA. The expression cassette can be used for constructing an expression vector or introducing a foreign gene into genomic DNA. Preferably, the expression cassette according to the present invention is a DNA construct.

As used herein, the term "heterologous" pertaining to polynucleotides of genes and control regions means that two elements are not derived from the same polynucleotide chain. For example, in the case where two polynucleotides are "heterologous" to each other, they are derived from different polynucleotide chains regardless of whether the polynucleotides are derived from genes with the same name or derived from genes with different names. Therefore, in the case where a 5'-UTR of a promoter of a certain gene is substituted with a foreign DNA fragment, the 5'-UTR and the DNA fragment are "heterologous" to each other even if they are derived from genes with the same name, and the DNA fragment is heterologous to the promoter. Therefore, the term "heterologous" pertaining to polynucleotides includes the meaning that two elements are not derived from the same gene. In this case, when a 5'-UTR of a certain gene is substituted with a DNA fragment derived from a different gene, the 5'-UTR and the DNA fragment are "heterologous" to each other.

As used herein, the term *"Bacillus* bacteria" refers to bacteria belonging to the genus *Bacillus* in the family *Bacillaceae.* Examples of the *Bacillus* bacteria include *B. subtilis (Bacillus subtilis), B. cereus, B. thuringiensis, B. megaterium, B. amyloliquefaciens, B. pumilus, B. liqueniformis, B. licheniformis,* and variants thereof.

As used herein, the term "target substance" refers to any substance (for example, a peptide, a protein or a metabolic product) which is desired to be produced by host cells. As used herein, the term "target gene" refers to a polynucleotide encoding a target substance, or a substance which promotes a production process for the target substance (for example, biosynthesis or extracellular transport). For example, the target gene includes a gene encoding a protein or a peptide which is a target substance, a gene encoding a protein involved in biosynthesis of a metabolic product which is a target substance, and a gene encoding a protein involved in extracellular transport of a target substance. The target gene may be a heterologous gene encoding a heterologous expression product, an externally introduced gene of the same origin, a gene encoding an expression product which host cells originally have, or a gene encoding any other expression product (a protein, a peptide or the like).

Examples of the substance encoded by the target gene include enzymes, antibodies, insecticidal proteins, hormones, cytokines, physiologically active peptides, biosynthetic enzymes of metabolic products, transporters involved in extracellular release of metabolic products, and enzymes and insecticidal proteins are preferred. Examples of the enzyme include oxidoreductases, transferases, hydrolases, lyases, isomerases, and ligases or synthetases. Preferred examples include cellulose-based biomass degradation enzymes such as cellulase and hemicellulase, exoglucanase, endoglucanase, β-glucosidase, laccase, lactase, esterase, pectate lyase, pectinase, peroxidase, phytase, pullulanases, PETase, protease, lipase, mannanase, arabinase, galactase, and amylase, and more preferred examples include protease, lipase and amylase. Examples of the insecticidal protein include Cry proteins produced by *Bacillus thuringiensis.* Cry proteins classified as Cry4, Cry5 and Cry11 are preferred, Cry5B, Cry4Aa, Cry4Ba and Cry11Aa are more preferred, and Cry5B is still more preferred.

As used herein, the term "expression" of a gene includes both the process of transcription from a gene to mRNA and the process of translation of mRNA into a protein, and is properly used depending on the context. Basically, when the expression level of a target gene is improved, the amount of a target substance produced increases. The expression level of the target gene can be measured by gene expression analysis, or quantifying the amount of a protein produced and expressed from the target gene. For example, in comparison of the amounts of identical target substances produced in different cells, the concentrations of target substances produced may be compared, and in the case where the target substance is an enzyme, enzymic activity values may be compared. The enzymic activity value and the substance concentration are measured by methods well known to those skilled in the art. As used herein, the terms "improvement" and "promotion" can be used interchangeably when referring to the expression of a gene, and the terms "increase" and "improvement" can be used interchangeably when referring to the amount of a protein produced.

The names of genes or proteins described herein follow registration information in the Protein Data Bank (PDB) ([www.rcsb.org/]) unless otherwise specified.

Improvement of productivity in production of target substances by microorganisms is desired. The present invention relates to a DNA molecule containing a modified 5'-UTR which promotes the expression of a gene, or a promoter containing the modified 5'-UTR, and a method for producing a target substance using the same.

The present inventors found that by substituting at least a part of 5'-UTR contained in a promoter of a target gene in a microorganism with a specific DNA fragment derived from a 5'-UTR of a *Bacillus* endoglucanase gene, the expression of the target gene is remarkably promoted.

According to the present invention, target substance productivity with a microorganism can be remarkably improved.

The present invention provides a modified 5'-UTR, a modified promoter containing the modified 5'-UTR, and uses thereof in production of a target substance by a microorganism.

The modified 5'-UTR which is provided by the present invention can be constructed by modifying a 5'-UTR to be modified (hereinafter, also referred to as "parental 5'-UTR") using a heterologous polynucleotide. More specifically, the modified 5'-UTR of the present invention can be constructed by substituting a part or the whole of a parental 5'-UTR with a heterologous polynucleotide, or adding a heterologous polynucleotide to a parental 5'-UTR. Therefore, the parental 5'-UTR of the modified 5'-UTR is 5'-UTR which does not contain the heterologous polynucleotide.

The heterologous polynucleotide used for modification of the 5'-UTR is a polynucleotide heterologous to the parental 5'-UTR (that is, derived from a different polynucleotide chain). Preferably, the heterologous polynucleotide is a fragment derived from 5'-UTR of a *Bacillus* endoglucanase gene. Examples of the *Bacillus* endoglucanase gene include endoglucanase (for example, alkaline cellulase) genes of *Bacillus* sp. KSM-S237 strain (FERM BP-7875).

The heterologous polynucleotide contains the Shine-Dalgarno (SD) sequence GGAGG which functions as a ribosome binding site in a *Bacillus* gene. Preferably, the heterologous polynucleotide contains AGGAGG (SEQ ID NO: 1). More preferably, the heterologous polynucleotide contains AGGAGGTAATATG (SEQ ID NO: 2). Preferably, the heterologous polynucleotide has a length of at least 20 nt. The upper limit of the length of the heterologous polynucleotide is preferably 100 nt or less though it is not particularly limited.

Preferably, the heterologous polynucleotide contains the nucleotide sequence of WAWWTTWAGGAGGTAATATG (SEQ ID NO: 70, W is A or T), or a nucleotide sequence having at least 80% identity with the nucleotide sequence of SEQ ID NO: 70. However, such a heterologous polynucleotide contains AGGAGG (SEQ ID NO: 1) at positions corresponding to positions 8 to 13 of the nucleotide sequence of SEQ ID NO: 70. Preferably, such a heterologous polynucleotide contains AGGAGGTAATATG (SEQ ID NO: 2) at positions corresponding to positions 8 to 20 of the nucleotide sequence of SEQ ID NO: 70. In an embodiment, the heterologous polynucleotide consists of the nucleotide sequence of SEQ ID NO: 70. In an embodiment, the heterologous polynucleotide has a sequence derived from the 5'-UTR of a *Bacillus* endoglucanase gene adjacent to the 5'-end of the nucleotide sequence of SEQ ID NO: 70. For example, the heterologous polynucleotide consists of the nucleotide sequence of SEQ ID NO: 69, or a fragment consisting of the 3'-end region of a polynucleotide consisting of a sequence having at least 80% identity with the nucleotide sequence of SEQ ID NO: 69 (the 3'-end consists of the nucleotide sequence of SEQ ID NO: 70).

In an embodiment, the heterologous polynucleotide contains the nucleotide sequence of SEQ ID NO: 3. The nucleotide sequence of SEQ ID NO: 3 is derived from the 5'-UTR (SEQ ID NO: 69) of an alkaline cellulase gene of *Bacillus* sp. KSM-S237 (FERM BP-7875). In a preferred embodiment, the heterologous polynucleotide consists of the nucleotide sequence of SEQ ID NO: 3.

In another embodiment, the heterologous polynucleotide contains a nucleotide sequence having at least 80% identity with the nucleotide sequence of SEQ ID NO: 3. In a preferred embodiment, the heterologous polynucleotide consists of a nucleotide sequence having at least 80% identity with the nucleotide sequence of SEQ ID NO: 3. In another preferred embodiment, the heterologous polynucleotide consists of a nucleotide sequence obtained by deleting, substituting, adding or inserting one or several nucleotides in the nucleotide sequence of SEQ ID NO: 3. However, such a heterologous polynucleotide contains AGGAGG (SEQ ID NO: 1) at positions corresponding to positions 8 to 13 of the nucleotide sequence of SEQ ID NO: 3. Preferably, such a heterologous polynucleotide contains AGGAGGTAATATG (SEQ ID NO: 2) at positions corresponding to positions 8 to 20 of the nucleotide sequence of SEQ ID NO: 3.

In another embodiment, the heterologous polynucleotide contains the nucleotide sequence of SEQ ID NO: 4. The nucleotide sequence of SEQ ID NO: 4 is derived from the 5'-UTR (SEQ ID NO: 69) of an alkaline cellulase gene of *Bacillus* sp. KSM-S237 (FERM BP-7875), and contains the nucleotide sequence of SEQ ID NO: 3. In a preferred embodiment, the heterologous polynucleotide consists of the nucleotide sequence of SEQ ID NO: 4. In another preferred embodiment, the heterologous polynucleotide is a fragment (from 21 to 93 nt) of a polynucleotide consisting of the nucleotide sequence of SEQ ID NO: 4, which contains the nucleotide sequence of SEQ ID NO: 3.

In another embodiment, the heterologous polynucleotide contains a nucleotide sequence having at least 80% identity with the nucleotide sequence of SEQ ID NO: 4 or a fragment thereof. In a preferred embodiment, the heterologous polynucleotide consists of a nucleotide sequence having at least 80% identity with the nucleotide sequence of SEQ ID NO: 4. In another preferred embodiment, the heterologous polynucleotide consists of a nucleotide sequence obtained by deleting, substituting, adding or inserting one or several nucleotides in the nucleotide sequence of SEQ ID NO: 4 or a fragment thereof. However, such a heterologous polynucleotide contains AGGAGG (SEQ ID NO: 1) at positions corresponding to positions 8 to 13 of the nucleotide sequence of SEQ ID NO: 3. Preferably, such a heterologous polynucleotide contains AGGAGGTAATATG (SEQ ID NO: 2) at positions corresponding to positions 8 to 20 of the nucleotide sequence of SEQ ID NO: 3.

In another embodiment, the heterologous polynucleotide contains the nucleotide sequence of SEQ ID NO: 5. In a preferred embodiment, the heterologous polynucleotide consists of the nucleotide sequence of SEQ ID NO: 5. In another embodiment, the heterologous polynucleotide contains a nucleotide sequence having at least 80% identity with the nucleotide sequence of SEQ ID NO: 5. In a preferred embodiment, the heterologous polynucleotide consists of a nucleotide sequence having at least 80% identity with the nucleotide sequence of SEQ ID NO: 5. In another preferred embodiment, the heterologous polynucleotide consists of a nucleotide sequence obtained by deleting, substituting, adding or inserting one or several nucleotides in the nucleotide sequence of SEQ ID NO: 5. However, such a heterologous polynucleotide contains AGGAGG (SEQ ID NO: 1) at positions corresponding to positions 8 to 13 of the nucleotide sequence of SEQ ID NO: 5. Preferably, such a heterologous polynucleotide contains AGGAGGTAATATG (SEQ ID NO: 2) at a position corresponding to positions 8 to 20 of the nucleotide sequence of SEQ ID NO: 5.

In another embodiment, the heterologous polynucleotide contains the nucleotide sequence of SEQ ID NO: 6. In a preferred embodiment, the heterologous polynucleotide consists of the nucleotide sequence of SEQ ID NO: 6. In another embodiment, the heterologous polynucleotide contains a nucleotide sequence having at least 80% identity with the nucleotide sequence of SEQ ID NO: 6. In a preferred embodiment, the heterologous polynucleotide consists of a nucleotide sequence having at least 80% identity with the nucleotide sequence of SEQ ID NO: 6. In another preferred embodiment, the heterologous polynucleotide consists of a nucleotide sequence obtained by deleting, substituting, adding or inserting one or several nucleotides in the nucleotide sequence of SEQ ID NO: 6. However, such a heterologous polynucleotide contains AGGAGG (SEQ ID NO: 1) at positions corresponding to positions 8 to 13 of the nucleotide sequence of SEQ ID NO: 6. Preferably, such a heterologous polynucleotide contains AGGAGGTAATATG (SEQ ID NO: 2) at positions corresponding to positions 8 to 20 of the nucleotide sequence of SEQ ID NO: 6.

SEQ ID NO: 3 = AATATTTAGGAGGTAATATG
SEQ ID NO: 4 = 
GTTTTTTTAAAACTTTAACGAAAGCACTTTCGGTAATGCTTATGAATTTAGCTATTT

GATTCAATTACTTTAAAAATATTTAGGAGGTAATATG
SEQ ID NO: 5 = TAATTTTAGGAGGTAATATG
SEQ ID NO: 6 = TAATTTAAGGAGGTAATATG

The heterologous polynucleotide can be prepared by a gene engineering method known in the art. For example, the heterologous polynucleotide can be isolated from the genome of *Bacillus* bacteria (for example, KSM-S237) by a known method. Alternatively, a heterologous polynucleotide having a desired nucleotide sequence can be prepared by introducing a mutation into the isolated polynucleotide using a known method. A method for deleting, substituting, inserting or adding a nucleotide(s) in a nucleotide sequence is described in, for example, Dieffenbach et al, (Cold Spring Harbar Laboratory Press, New York, 581-621, 1995). Alternatively, the heterologous polynucleotide can be chemically synthesized. For the chemical synthesis of the polynucleotide, a commercially available DNA synthesis service is available.

The parental 5'-UTR to be modified with the heterologous polynucleotide is preferably the 5'-UTR contained in a promoter of a *Bacillus* gene. The promoter is preferably a high-functional promoter which exhibits high transcription promoting activity in *Bacillus* bacteria. Examples of the promoter include a promoter of a gene selected from the group consisting of a gene encoding a secreted protein of *Bacillus* bacteria, for example, an endoglucanase gene of *Bacillus* sp. KSM-64, an endoglucanase gene of *Bacillus* sp. KSM-S237, aprE, nprE and amyE of *Bacillus subtilis,* and amyL and amyQ of *B. licheniformis;* a promoter of the Bacillus subtilis spoVG gene; a promoter of a gene selected from the group consisting of ribosome RNA genes and ribosome-related genes, for example, rrnO, rrnE, rrnI, rrnJ, rrnB, rpsD, rpsJ and rpoD; a promoter of a cry-crystalline protein gene of *B. thuringensis,* for example, a cryIIIA gene; and a P43 promoter, a SP82 promoter or a scr promoter of *Bacillus* bacteria. However, the parental 5'-UTR does not have the sequence of SEQ ID NO: 70.

Preferably, the parental 5'-UTR is derived from a promoter of a gene differing from a gene from which the heterologous polynucleotide is derived. For example, in the case where the heterologous polynucleotide contains any of the nucleotide sequences of SEQ ID NOS: 3 to 6, it is preferred that the parental 5'-UTR be not the 5'-UTR of the alkaline cellulase gene of *Bacillus* sp. KSM-S237.

The parental 5'-UTR may exist in a genome inside the cell, may exist in an isolated polynucleotide fragment (for example, an isolated fragment of a region containing a promoter), or may be contained in a vector such as an expression vector. For example, a heterologous polynucleotide can be directly substituted for or added to the parental 5'-UTR in a genome inside the cell by a homologous recombination method or the like. Alternatively, a heterologous polynucleotide is substituted for or added to the parental 5'-UTR in a promoter fragment isolated from a genome, or parental 5'-UTR on an expression vector, whereby a modified 5'-UTR can be constructed.

The heterologous polynucleotide can be substituted for or added to the parental 5'-UTR by a usual method in the art. For example, a fragment containing a parental 5'-UTR and a fragment containing the heterologous polynucleotide are amplified by PCR and the like, and the resulting fragments are linked by SOE (splicing by overlap extension)-PCR (Gene, 1989, 77 (1): p 61-68), whereby a modified 5'-UTR can be constructed. Alternatively, a modified 5'-UTR can be constructed by inverse PCR using a primer designed to encode the polynucleotide of the modified 5'-UTR and a vector containing the heterologous polynucleotide as a template.

In a preferred embodiment, the resulting modified 5'-UTR contains the heterologous polynucleotide at the 3'-end. That is, the heterologous polynucleotide is added to the 3'-end of parental 5'-UTR, or the 3'-end of the parental 5'-UTR is substituted with the heterologous polynucleotide. Preferably, the modified 5'-UTR is constructed by substituting the 3'-end of the parental 5'-UTR with the heterologous polynucleotide, and has lost one or more nucleotides at the 3'-end of the parental 5'-UTR. The length of the 3'-end portion of the parental 5'-UTR, which is substituted with the heterologous polynucleotide, is not particularly limited, and is, for example, 20 nt or more. At the maximum, the whole of the parental 5'-UTR may be substituted.

In a preferred embodiment, the modified 5'-UTR is constructed by substituting at least 20 nucleotides at the 3'-end (1 to 20 nucleotides from the 3'-end) of the parental 5'-UTR with the heterologous polynucleotide, and does not contain at least 20 nucleotides at the 3'-end of the parental 5'-UTR.

In another preferred embodiment, the modified 5'-UTR is constructed by substituting at least 26 nucleotides at the 3'-end (1 to 26 nucleotides from the 3'-end) of the parental 5'-UTR with the heterologous polynucleotide, and does not contain at least 26 nucleotides at the 3'-end of the parental 5'-UTR.

In another preferred embodiment, the modified 5'-UTR is constructed by substituting a region from the SD sequence (that is, a sequence consisting of GGAGG) to the 3'-end of the parental 5'-UTR with the heterologous polynucleotide, and does not contain the region from the SD sequence to the 3'-end of the parental 5'-UTR.

In another preferred embodiment, the modified 5'-UTR is constructed by substituting a region from a sequence consisting of AGGAGG (SEQ ID NO: 1) to the 3'-end in the parental 5'-UTR with the heterologous polynucleotide, and does not contain the region from the sequence consisting of SEQ ID NO: 1 to the 3'-end in the parental 5'-UTR.

In another preferred embodiment, the modified 5'-UTR consists of the heterologous polynucleotide. That is, the whole of the parental 5'-UTR is substituted with the heterologous polynucleotide.

The modified 5'-UTR obtained in the present invention may contain the SD sequence of the parental 5'-UTR though it is not required to contain the SD sequence of the parental 5'-UTR. The length of the modified 5'-UTR obtained in the present invention is not particularly limited as long as a gene expression improving effect can be exhibited.

In a further embodiment of the present invention, the modified 5'-UTR may further contain at least one additional ribosome binding sequence (RBS) (that is, heterologous RBS). The heterologous RBS is arranged upstream of the SD sequence GGAGG contained in the modified 5'-UTR. Preferably, the heterologous RBS is arranged upstream of the region consisting of AGGAGG (SEQ ID NO: 1) contained in the modified 5'-UTR. More preferably, the heterologous RBS is arranged 8 or more nucleotides upstream of the region consisting of AGGAGG (SEQ ID NO: 1) contained in the modified 5'-UTR. Still more preferably, the heterologous RBS is arranged upstream of the heterologous polynucleotide contained in the modified 5'-UTR. In the case where two or more of the heterologous RBSs exist, all of them are arranged upstream of the SD sequence contained in the modified 5'-UTR, preferably upstream of the region consisting of SEQ ID NO: 1, more preferably 8 or more nucleotides upstream of the region consisting of SEQ ID NO: 1, and still more preferably upstream of the heterologous polynucleotide. In the case where the modified 5'-UTR contains two or more SD sequences, for example, the SD sequence derived from the parental 5'-UTR, and the heterologous polynucleotide, the heterologous RBS is arranged upstream of all of the SD sequences and the heterologous polynucleotide.

As used herein, the phrase that RBS is "arranged upstream of a certain region (or sequence)" means that the RBS is arranged such that the 3'-end of the RBS is located upstream of the 5'-end of the region (or sequence). As used herein, the phrase that RBS is "arranged N nucleotides upstream of a certain region (or sequence)" means that the RBS is arranged such that the 3'-end of the RBS is located N nucleotides upstream with respect to the 5'-end of the region (or sequence).

In an embodiment, the modified 5'-UTR does not contain the SD sequence derived from the parental 5'-UTR, and the heterologous RBS is arranged 8 or more nucleotides upstream of the AGGAGG (SEQ ID NO: 1) region derived from the heterologous polynucleotide contained in the modified 5'-UTR. In the case where two or more of the heterologous RBSs exist, all of them are arranged 8 or more nucleotides upstream of the AGGAGG (SEQ ID NO: 1) region derived from the heterologous polynucleotide.

In another embodiment, the modified 5'-UTR does not contain the SD sequence derived from the parental 5'-UTR, and the heterologous RBS is arranged upstream of the heterologous polynucleotide contained in the modified 5'-UTR. In the case where two or more of the heterologous RBSs exist, all of them are arranged upstream of the heterologous polynucleotide.

The heterologous RBS is a polynucleotide containing the nucleotide sequence of AGGAGG (SEQ ID NO: 1). Examples of the heterologous RBS include a polynucleotide consisting of the nucleotide sequence of SEQ ID NO: 1, a polynucleotide consisting of any of the nucleotide sequences of SEQ ID NOS: 7 to 11, and a polynucleotide consisting of a nucleotide sequence which is obtained by deleting, substituting, adding or inserting one or several nucleotides in the nucleotide sequence of any of SEQ ID NOS: 1 and 7 to 11 and which contains AGGAGG (SEQ ID NO: 1).
SEQ ID NO: 7 = AGGAGGGA
SEQ ID NO: 8 = GAAAGGAGG
SEQ ID NO: 9 = GAAAGGAGGGA
SEQ ID NO: 10 = CGAAAGGAGGGAT
SEQ ID NO: 11 = CTTGAAAGGAGGGATGCCTAA

At least one of the heterologous RBSs is located 10 to 100 nucleotides downstream, preferably 10 to 60 nucleotides downstream, and more preferably 13 to 55 nucleotides downstream of a transcription start site which is located upstream of the modified 5'-UTR (or a transcription start site of a modified promoter containing the modified 5'-UTR, which will be described later). In the case where two or more heterologous RBSs exist, the insertion position for the remaining RBS(s) is not particularly limited, preferably located 10 to 150 nucleotides downstream, more preferably 10 to 100 nucleotides downstream, still more preferably 10 to 60 nucleotides downstream, and still more preferably 13 to 55 nucleotides downstream of the transcription start site. As used herein, the phrase "RBS is located N nucleotides downstream of the transcription start site" means that the 5'-end of RBS is located N nucleotides downstream of the 3'-end of the transcription start site.

The modified 5'-UTR containing the heterologous RBS can be constructed by substituting, inserting or adding the polynucleotide of the heterologous RBS in the modified 5'-UTR containing the heterologous polynucleotide. For example, the polynucleotide of the heterologous RBS may be substituted, inserted or added upstream of the SD sequence or the heterologous polynucleotide contained in the modified 5'-UTR. Alternatively, by substituting, inserting or adding the polynucleotide of the heterologous RBS in the parental 5'-UTR, and then substituting or adding the heterologous polynucleotide downstream thereof, the modified 5'-UTR containing the heterologous RBS can be constructed. Alternatively, by substituting a fragment containing the polynucleotide of the heterologous RBS and the heterologous polynucleotide in this order from the upstream for a part or the whole of the parental 5'-UTR, the modified 5'-UTR containing heterologous RBS can be constructed. The procedure for substituting, inserting or adding the polynucleotide of heterologous RBS in the parental 5'-UTR or modified 5'-UTR can be carried out by a known method such as the above-described SOE-PCR or inverse PCR.

The modified 5'-UTR obtained in the present invention serves to improve the expression of a gene which is controlled by a promoter containing the modified 5'-UTR. Specifically, the modified 5'-UTR is located downstream of the transcription start site in the promoter and upstream of ORF of the gene, and is operably linked to the ORF of the gene.

In a preferred embodiment, the modified promoter containing the modified 5'-UTR is produced according to the present invention. The modified promoter is produced by modifying the 5'-UTR contained in a promoter containing the parental 5'-UTR (a parent promoter) which does not contain the heterologous polynucleotide. The 5'-UTR is modified by substituting the heterologous polynucleotide for a part or the whole of the 5'-UTR, or adding the heterologous polynucleotide to the 5'-UTR. The modification of the 5'-UTR may further include substituting, inserting or adding a heterologous RBS in the 5'-UTR. The heterologous polynucleotide and the heterologous RBS to be used for the modification, and the method for substituting, inserting or adding the heterologous polynucleotide or heterologous RBS in the 5'-UTR are as described above. Therefore, the heterologous polynucleotide and heterologous RBS contained in the modified promoter are heterologous to the parent promoter (derived from a different polynucleotide chain). Preferably, the heterologous polynucleotide contained in the modified promoter is derived from a gene differing from the parent promoter.

The parent promoter to be subjected to the modification may be a promoter derived from a control region of any gene, and is preferably a promoter of a *Bacillus* gene. The parent promoter is preferably a high-function promoter which can exhibit high transcription promoting activity in *Bacillus* bacteria. Preferred examples of the parent promoter include a promoter of a gene selected from the group consisting of a gene encoding a secreted protein of *Bacillus* bacteria, for example, an endoglucanase gene of *Bacillus* sp. KSM-64, an endoglucanase gene of *Bacillus* sp. KSM-S237, aprE, nprE and amyE of *Bacillus subtilis,* and amyL and amyQ of *B. licheniformis;* a promoter of the *Bacillus subtilis* spoVG gene; a promoter of a gene selected from the group consisting of ribosome RNA genes and ribosome-related genes, for example, rrnO, rrnE, rrnI, rrnJ, rrnB, rpsD, rpsJ and rpoD; a promoter of a cry-crystalline protein gene of *B. thuringensis,* for example, a cryIIIA gene; and a P43 promoter, a SP82 promoter, a scr promoter, and a modified promoter derived therefrom. Preferred examples include a promoter derived from the alkaline cellulase gene of *Bacillus* sp. KSM-64, which is set forth as SEQ ID NO: 72, and a promoter of the *Bacillus subtilis* spoVG gene, which is set forth as SEQ ID NO: 76. Another preferred example of the parent promoter includes a polynucleotide which consists of a nucleotide sequence having at least 80% identity with the nucleotide sequence of SEQ ID NO: 72, and has a function of controlling the expression of a gene, or a polynucleotide which consists of a nucleotide sequence having at least 80% identity with the nucleotide sequence of SEQ ID NO: 76, and has a function of controlling the expression of a gene. The function of controlling the expression of a gene by a promoter polynucleotide can be measured by a known method.

The parent promoter may exist in a genome inside the cell, may be an isolated polynucleotide fragment, or may be contained in a vector such as an expression vector.

For example, a polynucleotide fragment of a parent promoter is prepared from *Bacillus* bacteria, a heterologous polynucleotide is substituted for a part or the whole of the 5'-UTR contained in the parent promoter, or the heterologous polynucleotide is added to the 5'-UTR, whereby a fragment of a modified promoter can be constructed. Alternatively, a 5'-UTR contained in a parent promoter in a genome of *Bacillus* bacteria is modified by substituting a heterologous polynucleotide substituted for a part or the whole of the 5'-UTR or adding the heterologous polynucleotide to the 5'-UTR by a homologous recombination method or the like, whereby a modified promoter can be constructed in the genome. Alternatively, a promoter in a genome of *Bacillus* bacteria is substituted with a modified promoter containing the modified 5'-UTR by a homologous recombination method, whereby a modified promoter can be constructed in the genome.

The modified 5'-UTR, when it is arranged in a promoter, serves to improve the expression of a gene. The modified promoter containing the modified 5'-UTR can improve the expression of a gene as compared to a parent promoter containing a parental 5'-UTR before modification (that is, a promoter before it is modified so that the modified 5'-UTR is contained therein). Preferably, the modified promoter containing the modified 5'-UTR increases the expression level of the gene by at least 10%, preferably by at least 20%, more preferably by at least 25%, and still more preferably by at least 30% as compared to the parent promoter. In the case where the modified 5'-UTR contains the above-described heterologous RBS, the modified promoter can further improve the expression level of the gene, and preferably increases the expression level of the gene by at least 40%, more preferably by at least 50%, still more preferably by at least 100%, still more preferably by at least 200%, still more preferably by at least 300%, and still more preferably by at least 400% as compared to the parent promoter.

The modified 5'-UTR and the modified promoter containing the modified 5'-UTR can be used for improving the expression of a target gene. Therefore, the present invention provides a polynucleotide containing the modified 5'-UTR, or a modified promoter containing the modified 5'-UTR, and use thereof for expression of a target gene. Preferably, the polynucleotide is DNA.

In an embodiment, the polynucleotide containing the modified 5'-UTR or modified promoter can be directly introduced into the genome of the host cell. For example, the polynucleotide containing the modified 5'-UTR or modified promoter can be introduced into the genome of the host cell, and operably linked to the target gene.

In an embodiment, the polynucleotide containing the modified 5'-UTR or modified promoter is an expression cassette. The expression cassette may contain, in addition to the modified 5'-UTR or modified promoter, a cis-element which improves the transcriptional activity of the promoter or 3'-UTR and the like. Further, the expression cassette may contain selection marker genes such as a drug-resistance gene and auxotrophic marker gene.

In an embodiment, the polynucleotide containing the modified 5'-UTR or modified promoter further contains a target gene. The target gene is operably linked to the modified 5'-UTR or modified promoter.

In an embodiment, the expression cassette is an expression vector. For example, the polynucleotide containing the modified 5'-UTR or modified promoter is conventionally introduced into any vector, whereby the expression vector can be produced. For example, the polynucleotide is constructed such that it has a restriction enzyme recognition sequence at both end thereof. By incorporating this into an expression vector cleaved by a restriction enzyme, the expression vector according to the present invention can be constructed (restriction enzyme method). Preferably, the modified 5'-UTR or modified promoter is operably linked upstream of the polynucleotide of the target gene in the expression vector.

The type of vector is not particularly limited. Any vector may be used such as a plasmid, a phage, a phagemid, a cosmid, a virus, an YAC vector or a shuttle vector. The vector may be intended to be introduced into the genome of the host cell, or may be retained outside the genome. The vector can be amplified preferably in bacteria, and more preferably in *Bacillus* bacteria (for example, *Bacillus subtilis* or variants thereof). Preferably, the vector is an expression vector capable of inducing the expression of an introduced gene in *Bacillus* bacteria.

Examples of the vector include pBluescript II SK(-) (Stratagene), pUC vectors such as pUC18/19 and pUC118/119 (Takara Bio Inc.), pET vectors (Takara Bio Inc.), pGEX vectors (GE Healthcare Technologies Inc.), pCold vector (Takara Bio Inc.), pHY300PLK (Takara Bio Inc.), pUB110 (Plasmid, 1986, 15(2): 93-103), pBR322 (Takara Bio Inc.), pRS403 (Stratagene), pMW vectors such as pMW118/119 and pMW218/21 (Nippon Gene Co., Ltd.), pRI vectors such as pRI909/910 (Takara Bio Inc.), pBI vectors (Clontech Laboratories, Inc.), IN3 vectors (Inplanta Innovations Inc.), pPTR1/2 (Takara Bio Inc.), pDJB2 (Gene, 1985, 36: 321-331), pAB4-1 (Mol Gen Genet, 1987, 206: 71-75), pLeu4 (Gene, 1989, 84: 335-343), pPyr225 (Mol Genet Genomics, 2002, 268: 397-406), pFG1 (Curr Genet, 1990, 18: 447-451), yeast expression vector pNAN8142 (Biosci Biotechnol Biochem, 1996, 60: 383-389), and pMA91 (Biosci Biotechnol Biochem, 1998, 62: 1615-1618).

The present invention also provides a transformant which contains a polynucleotide containing the modified 5'-UTR or modified promoter. The transformant can be produced by introducing the polynucleotide containing the modified 5'-UTR or modified promoter into host cells.

Examples of the host for the transformant include microorganism cells, preferably *Bacillus* bacteria, *Clostridium* bacteria, yeast, and *Escherichia coli.* Among them, *Bacillus* bacteria are preferred, and *Bacillus subtilis* or variants thereof are more preferred. Therefore, the transformant of the present invention is preferably recombinant *Bacillus* bacteria, and more preferably a recombinant of *Bacillus subtilis* or a variant thereof.

For introducing the polynucleotide containing the modified 5'-UTR or modified promoter into host cells, a well-known transformation method such as a calcium phosphate method, an electroporation method, a lipofection method, a particle gun method or a PEG method can be applied. Examples of the method applicable to *Bacillus subtilis* or a variant thereof include a competent cell transformation method (J Bacteriol, 1967, 93: 1925-1937), an electroporation method (FEMS Microbiol Lett, 1990, 55: 135-138), a protoplast transformation method (Mol Gen Genet, 1979, 168: 111-115), and a Tris-PEG method (J Bacteriol, 1983, 156: 1130-1134).

The transformant can be used for expression of a target gene. In the cells of the transformant, the modified 5'-UTR or modified promoter increases the expression of a target gene operably linked thereto, whereby the productivity of the target substance with the transformant is improved. For example, transformant cells which contain an expression cassette containing a modified promoter containing the modified 5'-UTR, and a target gene increase the expression level of the target gene preferably by at least 10%, more preferably by at least 20%, and still more preferably by at least 30% when the expression level of the target gene in cells containing an expression cassette containing a parent promoter and the target gene is assumed to be 100%. Further, in the case where the modified 5'-UTR contains the above-described heterologous RBS, the expression level of the target gene can be further improved. For example, transformant cells which contain an expression cassette containing the modified promoter containing modified 5'-UTR containing heterologous RBS, and a target gene increase the expression level of the target gene preferably by at least 50%, more preferably by at least 100%, still more preferably by at least 200%, still more preferably by at least 300%, and still more preferably by at least 400% when the expression level of the target gene in cells containing an expression cassette containing a parent promoter and the target gene is assumed to be 100%.

Therefore, the present invention also provides a method for producing a target substance, including culturing the transformant. The transformant can be cultured by a common method in the art. For example, in the case where the transformant is *Bacillus subtilis* or a variant thereof, the medium for culturing the cells can contain carbon sources necessary for growth of *Bacillus subtilis,* and inorganic nitrogen sources or organic nitrogen sources. If necessary, the medium may contain other nutrients, for example, inorganic salts, vitamins and antibiotics. Culture conditions, for example, the temperature, the aeration and stirring conditions, the pH of the medium, and the culture time can be appropriately selected according to the type and characteristics of the microorganism, the culture scale, and the like.

After the culturing, the target substance can be collected from the culture. If necessary, the collected target substance may be further purified. The method for collecting or purifying the target substance from the culture is not particularly limited. A known collection or purification method may be used. For example, the culture is collected, cell crushing treatment with ultrasonic waves, pressure or the like is performed if necessary, cell components are then removed by a gradient method, filtration, centrifugation or the like, and fractions containing the remaining target substance is then collected. Alternatively, a polynucleotide encoding a secretion signal peptide which functions in the transformant is operably linked to a gene encoding the target substance, whereby the target substance can be secreted outside the cell, and produced. In this case, fractions containing the target substance can be collected without crushing the cells.

If necessary, the collected fractions containing the target substance can be subjected to any of methods such as dialysis, salting-out, an ion exchange method, distillation and solvent extraction, or a combination thereof to purify the target substance. In the method for producing a target substance according to the present invention, culturing of the transformant and collection of the target substance may be performed batch-wise, semibatch-wise or continuously.

As illustrative embodiments of the present invention, the following substances, production methods, uses, methods and the like are disclosed herein, but the present invention is not limited to these embodiments.

[1] A DNA molecule comprising a modified promoter,
   the modified promoter comprising a modified 5'-UTR containing a heterologous polynucleotide,
   the heterologous polynucleotide comprising a nucleotide sequence of SEQ ID NO: 70, or a nucleotide sequence having at least 80% identity with the nucleotide sequence of SEQ ID NO: 70.
[2] The DNA molecule according to [1], wherein preferably, the heterologous polynucleotide is any of the following polynucleotides a), c) and d):
   a) a polynucleotide comprising a nucleotide sequence of SEQ ID NO: 3, or a nucleotide sequence having at least 80% identity with the nucleotide sequence of SEQ ID NO: 3;
   c) a polynucleotide comprising a nucleotide sequence of SEQ ID NO: 5, or a nucleotide sequence having at least 80% identity with the nucleotide sequence of SEQ ID NO: 5; and
   d) a polynucleotide comprising a nucleotide sequence of SEQ ID NO: 6, or a nucleotide sequence having at least 80% identity with the nucleotide sequence of SEQ ID NO: 6.
[3] The DNA molecule according to [2], wherein preferably, the polynucleotide a) comprises the following:
   b) a polynucleotide consisting of a nucleotide sequence of SEQ ID NO: 4, or a nucleotide sequence having at least 80% identity with the nucleotide sequence of SEQ ID NO: 4, or a fragment thereof.
[4] The DNA molecule according to any one of [1] to [3],
   wherein the heterologous polynucleotide preferably comprises a nucleotide sequence of SEQ ID NO: 1 at positions corresponding to positions 8 to 13 of the nucleotide sequence of SEQ ID NO: 70, and
   more preferably comprises the nucleotide sequence of SEQ ID NO: 1 at positions corresponding to positions 8 to 13 of the nucleotide sequence of SEQ ID NO: 3, or positions corresponding to positions 8 to 13 of the nucleotide sequence of SEQ ID NO: 5 or 6, or
   preferably comprises a nucleotide sequence of SEQ ID NO: 2 at positions corresponding to positions 8 to 20 of the nucleotide sequence of SEQ ID NO: 70, and
   more preferably comprises the nucleotide sequence of SEQ ID NO: 2 at positions corresponding to positions 8 to 20 of the nucleotide sequence of SEQ ID NO: 3, or positions corresponding to positions 8 to 20 of the nucleotide sequence of SEQ ID NO: 5 or 6.
[5] The DNA molecule according to any one of [1] to [4], wherein preferably, the heterologous polynucleotide has a length of 100 nt or less.
[6] The DNA molecule according to any one of [1] to [5], wherein preferably, the modified 5'-UTR has a DNA sequence obtained by substituting a part or a whole of a 5'-UTR before modification with the heterologous polynucleotide, or a DNA sequence obtained by adding the heterologous polynucleotide to the 5'-UTR before modification.
[7] The DNA molecule according to any one of [1] to [6], wherein preferably, the heterologous polynucleotide is located at the 3'-end of the modified 5'-UTR.
[8] The DNA molecule according to [7], wherein preferably, the modified 5'-UTR does not contain at least 20 nucleotides at the 3'-end of 5'-UTR before modification,
   does not contain at least 3'-terminus 26 nucleotides at the 3'-end of 5'-UTR before modification, or
   does not contain a region from a Shine-Dalgarno (SD) sequence to the 3'-end of 5'-UTR before modification.
[9] The DNA molecule according to any one of [1] to [8], wherein preferably, the modified 5'-UTR comprises a heterologous ribosome binding sequence in addition to the heterologous polynucleotide, and the ribosome binding sequence is arranged upstream of a region consisting of the nucleotide sequence of SEQ ID NO: 1 contained in the modified 5'-UTR.
[10] The DNA molecule according to [9], wherein preferably, the heterologous ribosome binding sequence is arranged upstream of the heterologous polynucleotide.
[11] The DNA molecule according to [9] or [10], wherein preferably, the modified 5'-UTR comprises one heterologous ribosome binding sequence, and the heterologous ribosome binding sequence is located from 10 to 100 nucleotides downstream of a transcription start site of the modified promoter, or
   the modified 5'-UTR comprises two or more heterologous ribosome binding sequences, at least one of the two or more heterologous ribosome binding sequences is located from 10 to 100 nucleotides downstream of the transcription start site of the modified promoter, and the remaining heterologous ribosome binding sequences are located from 10 to 150 nucleotides downstream of the transcription start site.
[12] The DNA molecule according to any one of [1] to [11], wherein preferably, a parent promoter of the modified promoter is derived from *Bacillus* bacteria.
[13] The DNA molecule according to [12], wherein preferably, the parent promoter is a polynucleotide consisting of a nucleotide sequence of SEQ ID NO: 72, or a nucleotide sequence having at least 80% identity with the sequence of SEQ ID NO: 72, or a polynucleotide consisting of a nucleotide sequence of SEQ ID NO: 76, or a nucleotide sequence having at least 80% identity with the sequence of SEQ ID NO: 76.
[14] The DNA molecule according to any one of [1] to [13], wherein preferably, the modified promoter consists of nucleotide sequences of SEQ ID NOS: 73, 74, 75, 77, 78, 79 and 80.
[15] The DNA molecule according to any one of [1] to [14], wherein preferably, the DNA molecule further comprises a target gene, and
   the target gene is a polynucleotide encoding a target substance, or a substance involved in biosynthesis or extracellular transport of the target substance.
[16] The DNA molecule according to [15], wherein the target substance is preferably an enzyme or an insecticidal protein, and
   more preferably protease, lipase, amylase or Cry5B.
[17] The DNA molecule according to [15] or [16], which is preferably an expression cassette or an expression vector.
[18] A transformant comprising the DNA molecule according to any one of [1] to [17].
[19] The transformant according to [18], wherein the transformant comprises the DNA molecule according to [17], wherein the DNA molecule is an expression cassette which contains the modified promoter and further contains a target gene, and
   when an expression level of the target gene in cells comprising an expression cassette which contains a parent promoter of the modified promoter and further contains the target gene is assumed to be 100%,
   the transformant increases the expression level of the target gene preferably by at least 10%, more preferably by at least 20%, still more preferably by at least 30%, still more preferably by at least 50%, still more preferably by at least 100%, still more preferably by at least 200%, still more preferably by at least 300%, and still more preferably by at least 400%.
[20] The transformant according to [18] or [19], which is preferably *Bacillus* bacteria.
[21] A method for producing a target substance, comprising culturing the transformant according to any one of [18] to [20].
[22] A method for producing a modified promoter,
   the method comprising modifying a 5'-UTR contained in a parent promoter,
   wherein the modifying a 5'-UTR comprises substituting a part or a whole of the 5'-UTR with a heterologous polynucleotide, or adding the heterologous polynucleotide to the 5'-UTR,
   the heterologous polynucleotide comprising a nucleotide sequence of SEQ ID NO: 70, or a nucleotide sequence having at least 80% identity with the nucleotide sequence of SEQ ID NO: 70,
   the parent promoter comprising the 5'-UTR which does not contain the heterologous polynucleotide.
[23] The method according to [22], wherein preferably, the heterologous polynucleotide is any of the following polynucleotides a), c) and d):
   a) a polynucleotide comprising a nucleotide sequence of SEQ ID NO: 3, or a nucleotide sequence having at least 80% identity with the nucleotide sequence of SEQ ID NO: 3;
   c) a polynucleotide comprising a nucleotide sequence of SEQ ID NO: 5, or a nucleotide sequence having at least 80% identity with the nucleotide sequence of SEQ ID NO: 5; and
   d) a polynucleotide comprising a nucleotide sequence of SEQ ID NO: 6, or a nucleotide sequence having at least 80% identity with the nucleotide sequence of SEQ ID NO: 6.
[24] The method according to [23], wherein preferably, the polynucleotide a) comprises the following:
   b) a polynucleotide consisting of a nucleotide sequence of SEQ ID NO: 4, or a nucleotide sequence having at least 80% identity with the nucleotide sequence of SEQ ID NO: 4, or a fragment thereof.
[25] The method according to any one of [22] to [24],
   wherein the heterologous polynucleotide preferably comprises a nucleotide sequence of SEQ ID NO: 1 at positions corresponding to positions 8 to 13 of the nucleotide sequence of SEQ ID NO: 70, and
   more preferably comprises the nucleotide sequence of SEQ ID NO: 1 at positions corresponding to positions 8 to 13 of the nucleotide sequence of SEQ ID NO: 3, or positions corresponding to positions 8 to 13 of the nucleotide sequence of SEQ ID NO: 5 or 6, or
   preferably comprises a nucleotide sequence of SEQ ID NO: 2 at positions corresponding to positions 8 to 20 of the nucleotide sequence of SEQ ID NO: 70, and
   more preferably comprises the nucleotide sequence of SEQ ID NO: 2 at positions corresponding to positions 8 to 20 of the nucleotide sequence of SEQ ID NO: 3, or positions corresponding to positions 8 to 20 of the nucleotide sequence of SEQ ID NO: 5 or 6.
[26] The method according to any one of [22] to [25], wherein preferably, the heterologous polynucleotide has a length of 100 nt or less.
[27] The method according to any one of [22] to [26], wherein preferably, the modifying a 5'-UTR comprises substituting the heterologous polynucleotide for the 5'-UTR or adding the heterologous polynucleotide to the 5'-UTR so as to locate the heterologous polynucleotide at the 3'-end of a 5'-UTR after modification.
[28] The method according to [27], wherein preferably, the modifying a 5'-UTR comprises substituting at least 20 nucleotides at the 3'-end of the 5'-UTR with the heterologous polynucleotide,
   comprises substituting at least 26 nucleotides T the 3'-end of the 5'-UTR with the heterologous polynucleotide, or
   comprises substituting a region from a Shine-Dalgarno (SD) sequence to the 3'-end in the 5'-UTR with the heterologous polynucleotide.
[29] The method according to any one of [22] to [28], wherein preferably, the modifying a 5'-UTR further comprises substituting, inserting or adding a ribosome binding sequence in the 5'-UTR, and the ribosome binding sequence is arranged upstream of a region consisting of the nucleotide sequence of SEQ ID NO: 1 contained in the 5'-UTR.
[30] The method according to [29], wherein preferably, the heterologous ribosome binding sequence is arranged upstream of the heterologous polynucleotide.
[31] The method according to [29] or [30], wherein preferably, the modifying a 5'-UTR comprises substituting, inserting or adding one ribosome binding sequence in the 5'-UTR, and the ribosome binding sequence is arranged from 10 to 100 nucleotides downstream of a transcription start site of the modified promoter, or
   the modifying a 5'-UTR comprises substituting, inserting or adding two or more ribosome binding sequences in the 5'-UTR, at least one of the two or more ribosome binding sequences is arranged from 10 to 100 nucleotides downstream of the transcription start site of the modified promoter, and the remaining heterologous ribosome binding sequences are located from 10 to 150 nucleotides downstream of the transcription start site.
[32] The method according to any one of [22] to [31], wherein preferably, the parent promoter is derived from *Bacillus* bacteria.
[33] The method according to [32], wherein preferably, the parent promoter is a polynucleotide consisting of a nucleotide sequence of SEQ ID NO: 72, or a nucleotide sequence having at least 80% identity with the sequence of SEQ ID NO: 72, or a polynucleotide consisting of a nucleotide sequence of SEQ ID NO: 76, or a nucleotide sequence having at least 80% identity with the sequence of SEQ ID NO: 76.

### Examples

Hereinafter, the present invention will be described in more detail by way of Examples. In the following Examples, and drawings, "a.u." means an arbitrary unit.

### Example 1: Construction of plasmid for introduction of KP43 protease expression cassette

A plasmid for introducing a protease expression cassette via homologous recombination into the amyE locus in the *B. subtilis* genome was constructed as follows.

A vector fragment was amplified by PCR using a forward primer (SEQ ID NO: 12) and a reverse primer (SEQ ID NO: 13) with a pMW119 plasmid as a template. Next, for the ORF sequence of the amyE gene, an insert fragment was amplified by PCR using a forward primer (SEQ ID NO: 14) and a reverse primer (SEQ ID NO: 15) with the *B*. *subtisli* 168 strain genome as a template. The vector fragment and the insert fragment were ligated by the In-Fusion reaction, and introduced via transformation into *Escherichia coli* to give a pMW-amy plasmid.

A vector fragment was amplified by PCR using a forward primer (SEQ ID NO: 16) and a reverse primer (SEQ ID NO: 17) with the pMW-amy plasmid as a template. Next, an insert fragment containing the ORF sequence of a spectinomycin resistance gene was amplified by PCR using a forward primer (SEQ ID NO: 18) and a reverse primer (SEQ ID NO: 19) with the kao119 strain genome (JP-B-6088282) as a template. The vector fragment and the insert fragment were ligated by the In-Fusion reaction, and introduced via transformation into *Escherichia coli* to give a pMW-amy-sp plasmid.

A vector fragment was amplified by PCR using the forward primer (SEQ ID NO: 16) and a reverse primer (SEQ ID NO: 20) with the pMW-amy-sp plasmid as a template. Next, the insert fragment was amplified by PCR using a forward primer (SEQ ID NO: 21) and a reverse primer (SEQ ID NO: 22) with P_{SP64}-TS43 (SEQ ID NO: 71) as a template. The insert fragment contained an expression cassette containing a synthetic promoter (P_{SP64}, SEQ ID NO: 72) derived from a promoter of an endoglucanase gene of *Bacillus* sp. KSM-64, and ORF of a gene encoding an alkaline protease (KP43 protease) derived from *Bacillus* sp. KSM-KP43 (FERM BP-6532). The vector fragment and the insert fragment were ligated by the In-Fusion reaction, and introduced via transformation into *Escherichia coli* to give a pMW-TS43 plasmid.

Example 2: Construction of plasmid for introduction of KP43 protease expression cassette containing modified 5'-UTR

The 5'-UTR (UTR^{SP64}) of the promoter (P_{SP64}) contained in the pMW-TS43 plasmid constructed in Example 1 was modified.
1) A vector fragment was amplified by PCR using a forward primer (SEQ ID NO: 23) and a reverse primer (SEQ ID NO: 24) with the pMW-TS43 plasmid as a template. Next, an insert fragment containing the sequence of SEQ ID NO: 4 was amplified by PCR using a forward primer (SEQ ID NO: 25) and a reverse primer (SEQ ID NO: 26) with pHY-YR288 (JP-A-2022-019601) as a template. The sequence of SEQ ID NO: 4 was a part fragment (94 nt) of 5'-UTR contained in the promoter of an endoglucanase gene derived from KSM-S237. The vector fragment and the insert fragment were ligated by the In-Fusion reaction, and introduced via transformation into *Escherichia coli* to give a pMW-UTR^{S237}-a-TS43 plasmid. The promoter contained in the PMW-UTR^{S237}-a-TS43 plasmid is referred to as P_{SP64}-UTR^{S237}-a.
2) Further, a fragment was constructed by inverse PCR using a forward primer (SEQ ID NO: 27) and a reverse primer (SEQ ID NO: 28) with pMW-TS43 as a template, and introduced via transformation into *Escherichia coli* to give a pMW-UTR^{S237}-b-TS43 plasmid. Further, a fragment was constructed by inverse PCR using a forward primer (SEQ ID NO: 29) and a reverse primer (SEQ ID NO: 30) with pMW-TS43 as a template, and introduced via transformation into *Escherichia coli* to give a pMW-UTR^{S237}-c-TS43 plasmid.

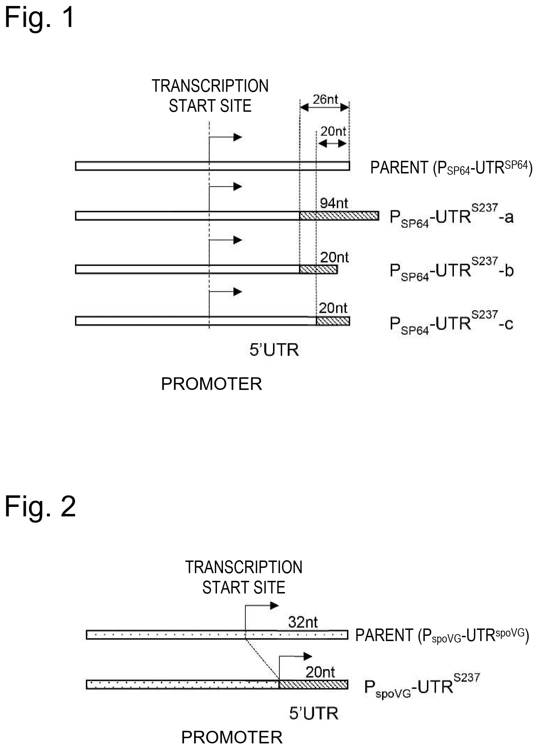

The promoters P_{SP64}-UTR^{S237}-a (SEQ ID NO: 73), P_{SP64}-UTR^{S237}-b (SEQ ID NO: 74) and P_{SP64}-UTR^{S237}-c (SEQ ID NO: 75) contained in pMW-UTR^{S237}-a-TS43 plasmid, pMW-UTR^{S237}-b-TS43 plasmid and pMW-UTR^{S237}-c-TS43 plasmid, respectively, each had the modified 5'-UTR shown in Fig. 1. As in Fig. 1, P_{SP64}-UTR^{S237}-a had the sequence of SEQ ID NO: 4 (shaded area) at the 3'-end of the 5'-UTR region, P_{SP64}-UTR^{S237}-b and P_{SP64}-UTR^{S237}-c each had the sequence of SEQ ID NO: 3 (shaded area) at the 3'-end of 5'-UTR.

### Example 3: Construction of plasmid for introduction of spoVG expression cassette containing modified 5'-UTR

The promoter of the pMW-TS43 plasmid constructed in Example 1 was substituted with the promoter of the spoVG gene of *B. subtisli* 168 strain (P_{spoVG}, SEQ ID NO: 76). A vector fragment was amplified by PCR using a forward primer (SEQ ID NO: 31) and a reverse primer (SEQ ID NO: 32) with the pMW-TS43 plasmid as a template. Next, an insert fragment containing the promoter of the spoVG gene of *B. subtisli* 168 strain (P_{spoVG}) was amplified by PCR using a forward primer (SEQ ID NO: 33) and a reverse primer (SEQ ID NO: 34) with the *B. subtisli* 168 strain genome as a template. The vector fragment and the insert fragment were ligated by the In-Fusion reaction, and introduced via transformation into *Escherichia coli* to give a pMW-P_{spoVG}TS43 plasmid.

The 5' -UTR region of P_{spoVG} (UTR^{spoVG}) in the pMW-P_{spoVG}-TS43 plasmid was modified. A fragment was constructed by inverse PCR using a forward primer (SEQ ID NO: 35) and a reverse primer (SEQ ID NO: 36) with pMW-P_{spoVG}-TS43 as a template, and introduced via transformation into *Escherichia coli* to give a pMW-P_{spoVG}-UTR^{S237}-TS43 plasmid. In the control region P_{spoVG}-UTR^{S237} (SEQ ID NO: 77) of the plasmid, the whole of the 5'-UTR of the parent promoter was substituted with the sequence of SEQ ID NO: 3 (shaded area) as shown in Fig. 2.

### Example 4: Construction of plasmid for introduction of amylase expression cassette containing modified 5'-UTR

A YR288 amylase expression cassette was constructed as follows.

A vector fragment was amplified by PCR using a forward primer (SEQ ID NO: 23) and a reverse primer (SEQ ID NO: 37) with the pMW-TS43 plasmid, which had been constructed in Example 1, as a template. Next, an insert fragment containing the ORF sequence of the YR288 amylase gene was amplified by PCR using a forward primer (SEQ ID NO: 38) and a reverse primer (SEQ ID NO: 39) with pHY-YR288 (JP-A-2022-019601) as a template. The vector fragment and the insert fragment were ligated by the In-Fusion reaction, and introduced via transformation into *Escherichia coli* to give a pMW-P_{SP64}-YR288 plasmid.

A vector fragment was amplified by PCR using a forward primer (SEQ ID NO: 23) and a reverse primer (SEQ ID NO: 40) with the pMW-UTR^{S237}-a-TS43 plasmid, which had been constructed in Example 2, as a template. Next, an insert fragment containing the ORF sequence of the YR288 amylase gene was amplified by PCR using a forward primer (SEQ ID NO: 41) and a reverse primer (SEQ ID NO: 39) with pHY-YR288 as a template. The vector fragment and the insert fragment were ligated by the In-Fusion reaction, and introduced via transformation into *Escherichia coli* to give a pMW-P_{SP64}-UTR^{S237}-a-YR288 plasmid.

A vector fragment was amplified by PCR using a forward primer (SEQ ID NO: 23) and a reverse primer (SEQ ID NO: 42) with the pMW-P_{spovG}-TS43, which had been constructed in Example 3, as a template. Next, an insert fragment containing the ORF sequence of the YR288 amylase gene was amplified by PCR using a forward primer (SEQ ID NO: 43) and a reverse primer (SEQ ID NO: 39) with pHY-YR288 as a template. The vector fragment and the insert fragment were ligated by the In-Fusion reaction, and introduced via transformation into *Escherichia coli* to give a pMW-P_{spoVG}-YR288 plasmid.

A vector fragment was amplified by PCR using a forward primer (SEQ ID NO: 23) and a reverse primer (SEQ ID NO: 44) with the pMW-P_{spoVG}-UTR^{S237}-TS43, which had been constructed in Example 3, as a template. Next, an insert fragment containing the ORF sequence of the YR288 amylase gene was amplified by PCR using a forward primer (SEQ ID NO: 41) and a reverse primer (SEQ ID NO: 39) with pHY-YR288 as a template. The vector fragment and the insert fragment were ligated by the In-Fusion reaction, and introduced via transformation into *Escherichia coli* to give a pMW-P_{spoVG}-UTR^{S237}-YR288 plasmid.

A fragment was constructed by inverse PCR using a forward primer (SEQ ID NO: 45) and a reverse primer (SEQ ID NO: 46) with the pMW-P_{spoVG}-UTR^{S237}-YR288 plasmid as a template, and introduced via transformation into *Escherichia coli* to give a pMW-P_{spoVG}-UTR^{S237}-mut1-YR288 plasmid. A fragment was constructed by inverse PCR using a forward primer (SEQ ID NO: 47) and a reverse primer (SEQ ID NO: 48) with pMW-P_{spoVG}-UTR^{S237}-YR288 as a template, and introduced via transformation into *Escherichia coli* to give a pMW-P_{spoVG}-UTR^{S237}-mut2-YR288 plasmid. The control regions P_{spoVG}-UTR^{S237}-mut1 (SEQ ID NO: 78) and P_{spoVG}-UTR^{S237}-mut2 (SEQ ID NO: 79) of the plasmids had the sequences of SEQ ID NO: 5 and 6, respectively, at the 3'-end of 5'-UTR.

### Example 5: Construction of plasmid for introduction of Cry5B expression cassette containing modified 5'-UTR

A Cry5B expression cassette was constructed. A vector fragment was amplified by PCR using a forward primer (SEQ ID NO: 23) and a reverse primer (SEQ ID NO: 37) with the pMW-TS43 plasmid, which had been constructed in Example 1, as a template. Next, an insert fragment containing the ORF sequence of the Cry5B gene was amplified by PCR using a forward primer (SEQ ID NO: 49) and a reverse primer (SEQ ID NO: 50) with pHY-Pscry5B (JP-B-7218090) as a template. The vector fragment and the insert fragment were ligated by the In-Fusion reaction, and introduced via transformation into *Escherichia coli* to give a pMW-P_{SP64}-cry5B plasmid.

A vector fragment was amplified by PCR using a forward primer (SEQ ID NO: 23) and a reverse primer (SEQ ID NO: 40) with the pMW-UTR^{S237}-a-TS43 plasmid, which had been constructed in Example 2, as a template. Next, an insert fragment containing the ORF sequence of the Cry5B gene was amplified by PCR using a forward primer (SEQ ID NO: 51) and a reverse primer (SEQ ID NO: 50) with pHY-Pscry5B as a template. The vector fragment and the insert fragment were ligated by the In-Fusion reaction, and introduced via transformation into *Escherichia coli* to give a pMW-P_{SP64}-UTR^{S237}-a-cry5B plasmid.

### Example 6: Construction of plasmid for introduction of lipase expression cassette containing modified 5'-UTR

A lipase expression cassette was constructed. A vector fragment was amplified by PCR using a forward primer (SEQ ID NO: 23) and a reverse primer (SEQ ID NO: 37) with the pMW-TS43 plasmid, which had been constructed in Example 1, as a template. Next, an insert fragment containing the ORF sequence of the lipase gene was amplified by PCR using a forward primer (SEQ ID NO: 53) and a reverse primer (SEQ ID NO: 54) with pHY-CnLip (SEQ ID NO: 52) as a template. The vector fragment and the insert fragment were ligated by the In-Fusion reaction, and introduced via transformation into *Escherichia coli* to give a pMW-P_{SP64}-Lip plasmid.

A vector fragment was amplified by PCR using a forward primer (SEQ ID NO: 23) and a reverse primer (SEQ ID NO: 40) with the pMW-UTR^{S237}-a-TS43 plasmid, which had been constructed in Example 2, as a template. Next, an insert fragment containing the ORF sequence of the lipase gene was amplified by PCR using a forward primer (SEQ ID NO: 55) and a reverse primer (SEQ ID NO: 54) with pHY-CnLip as a template. The vector fragment and the insert fragment were ligated by the In-Fusion reaction, and introduced via transformation into *Escherichia coli* to give a pMW-P_{SP64}-UTR^{S237}-a-Lip plasmid.

### Example 7: Addition of RBS to KP43 protease control region containing modified 5'-UTR

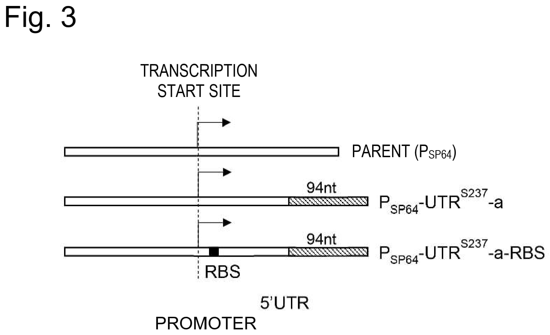

Studies were conducted on addition of a ribosome binding sequence (RBS) (CTTGAAAGGAGGGATGCCTAA; SEQ ID NO: 11) to a KP43 protease control region containing a modified 5'-UTR. A fragment was constructed by inverse PCR using a forward primer (SEQ ID NO: 56) and a reverse primer (SEQ ID NO: 57) with the pMW-P_{SP64}-UTR^{S237}-a-cry5B plasmid, which had been constructed in Example 5, as a template. The fragment was introduced via transformation into *Escherichia coli* to give PMW-P_{SP64}-UTR^{S237}-a-RBS-cry5B. As shown in Fig. 3, the control region P_{SP64}-UTR^{S237}-a-RBS (SEQ ID NO: 80) of the plasmid had RBS (black bar) added downstream of the transcription start site, and the sequence of SEQ ID NO: 4 at the 3'-end of 5'-UTR.

### Example 8: Production of recombinant Bacillus bacteria containing modified 5'-UTR

### 1) Construction of prsA overexpression strain

A prsA gene overexpression cassette (SEQ ID NO: 58) was introduced into a sigF gene deletion strain (ΔsigF strain: JP-B-4336082) of *B. subtilis* 168 strain to produce a prsA overexpression strain.

A DNA fragment for introduction of a prsA overexpression cassette was constructed by the following method. First, the following PCR fragments were constructed:
PCR fragment 1: forward primer; SEQ ID NO: 59, reverse primer; SEQ ID NO: 60, template DNA; prsA-Ka strain genome DNA (JP-B-4839144);
PCR fragment 2: forward primer; SEQ ID NO: 61, reverse primer; SEQ ID NO: 62), template DNA: MazF cassette (Genet. Syst., 84(4): 315-318, 2009);
PCR fragment 3: forward primer; SEQ ID NO: 63, reverse primer; SEQ ID NO: 64), template DNA: 168 strain genome DNA.

The PCR fragments 1 to 3 were ligated by SOE-PCR (forward primer; SEQ ID NO: 65, reverse primer; SEQ ID NO: 66) to construct a DNA fragment for introduction of a prsA overexpression cassette.

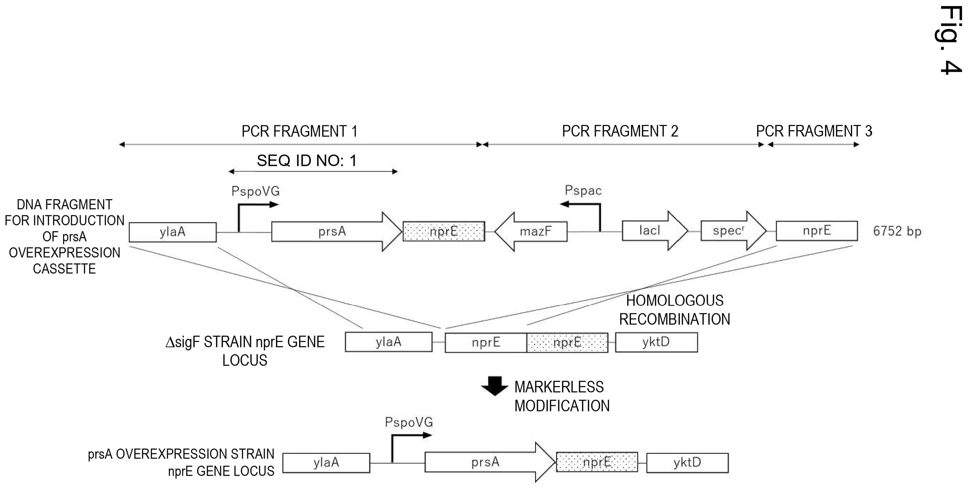

The constructed DNA fragment for introduction of a prsA overexpression cassette was introduced into the ΔsigF strain, and the prsA overexpression cassette was introduced into the nprE gene locus of the ΔsigF strain. The gene introduction was performed by homologous recombination using the marker-free deletion method developed by Morimoto et al. (Genet. Syst., 84(4): 315-318, 2009) (Fig. 4). The obtained prsA overexpression ΔsigF strain (ΔsigF-prsA strain) was used as a parent strain to produce recombinant *Bacillus* bacteria with the following target gene expression cassette.

### 2) Introduction of target gene expression cassette into parent strain genome

Protease and amylase expression strains were constructed by the following method. A PCR fragment for genome introduction was prepared by PCR using a forward primer (SEQ ID NO: 67) and a reverse primer (SEQ ID NO: 68) with the plasmid for introduction of a target gene expression cassette, which had been constructed in each of Examples 1 to 4 and 7, as a template. The PCR fragment was introduced into the ΔsigF-prsA strain produced as described in 1) above, by a competent cell method (see JP-B-6088282), and was introduced via transformation into the amyE gene locus on the genome by homologous recombination.

Cry5B and lipase expression strains were constructed by the following method. A PCR fragment for genome introduction was prepared by PCR using a forward primer (SEQ ID NO: 67) and a reverse primer (SEQ ID NO: 68) with the plasmid for introduction of a target gene expression cassette, which had been constructed in each of Examples 5 and 6, as a template. The PCR fragment was introduced into ΔsigF strain (JP-B-4336082) by the competent cell method (see JP-B-6088282), and introduced into the amyE gene locus on the genome by homologous recombination to produce recombinant *Bacillus* bacteria with an expression cassette.

### Example 9: Production of target protein by culturing recombinant Bacillus bacteria

The recombinant *Bacillus* bacteria with a target gene expression cassette, which had been obtained in Example 8, were cultured with shaking overnight at 180 rpm and 30°C in 2 mL of LB medium inside a 10 mL-volume roundbottomed centrifuge tube. 500 µL of the obtained culture solution was taken, and seeded in 20 mL of 2xL-maltose medium (2% peptone, 1% yeast extract, 1% NaCl, 7.5% maltose, 7.5 ppm manganese tetra-pentahydrate, defoaming agent; % denotes v/w%). This was cultured at 32°C (30°C for Cry5B expression strain and lipase expression strain) and 210 rpm for 72 hours.

### Example 10: Measurement of amount of target protein produced

### 1) Method

The amount of a target protein produced by recombinant *Bacillus* bacteria produced in Example 8 was measured.

The amount of protease produced was determined as a protease activity value. After the end of culture in Example 9, the protease activity of the culture supernatant from which the bacterial cells were removed was measured by the following procedure. 0.9 mL of a 1/15 M phosphate buffer solution (pH 7.4) and 0.05 mL of a 40 mM Glt-Ala-Ala-Pro-Leu-p-nitroanilide/dimethylsulfoxide solution were put in a test tube, and maintained at 30°C for 5 minutes. 0.05 mL of the culture supernatant was added thereto, the mixture was reacted at 30°C for 10 minutes, 2.0 mL of a 5% (w/v) aqueous citric acid solution was then added to stop the reaction, and an absorbance at 420 nm was measured using a spectrophotometer, and used as a protease activity value. The protease activity value of the recombinant *Bacillus* bacteria with an expression cassette containing the modified 5'-UTR was given as a relative value (a.u.) when the activity of recombinant *Bacillus* bacteria with an expression cassette containing the unmodified 5'-UTR is assumed to be 1.

The amount of amylase produced was determined as an amylase activity value. The amylase activity was measured by the following method. After the end of culture in Example 9, the amylase activity of the culture supernatant from which bacterial cells were removed was measured by the following procedure. 0.9 mL of an aqueous solution obtained by suspending one tablet of Phadebas Amylase Test in 5 mL of a 1/15 M phosphate buffer solution (pH 7.4) was added to a test tube, and maintained at 30°C for 15 minutes. 0.05 mL of an enzyme liquid (culture supernatant) diluted with a 2 mM aqueous calcium chloride solution as appropriate was added thereto, the mixture was reacted at 30°C accurately for 10 minutes, and 2 mL of a 5% (w/v) aqueous citric acid solution was then added to quench the reaction. The absorbance at 750 nm of a supernatant obtained by centrifuging the enzyme reaction solution at 3 000 rpm for 5 minutes was measured using a spectrophotometer, and used as an amylase activity value. The amylase activity value of the recombinant *Bacillus* bacteria with an expression cassette containing the modified 5'-UTR was given as a relative value (a.u.) when the activity of recombinant *Bacillus* bacteria with an expression cassette containing the unmodified 5'-UTR is assumed to be 1.

The amount of Cry5B produced was determined as a protein concentration. The Cry5B protein concentration was measured by a method described in JP-B-7218090. Specifically, after the end of culture in Example 9, the Cry5B concentration in the culture supernatant from which bacterial cells were removed was measured by the following procedure. SDS-PAGE was performed on the culture supernatant, and bovine serum albumin (BSA) (Wako Pure Chemical Industries, Ltd.) as a standard protein. The gel was stained with Bio-Safe^{™} Coomassie (Bio-Rad Laboratories, Inc.) while being shaken for 1 hour, followed by decoloring with ion-exchange water. The intensity of each band in the gel image was analyzed using image software ImageJ developed by the National Institutes of Health (rsb.info.nih.gov/ij/download.html). A calibration curve was prepared from data of BSA. From the calibration curve, the Cry5B protein concentration was calculated. The Cry5B protein concentration of recombinant *Bacillus* bacteria with an expression cassette containing the modified 5'-UTR was given as a relative value (a.u.) when the Cry5B protein concentration of recombinant *Bacillus* bacteria with an expression cassette containing the unmodified 5'-UTR is assumed to be 1.

The amount of lipase produced was determined as a lipase activity value. After the end of culture in Example 9, the lipase activity of the culture supernatant from which bacterial cells were removed was measured by the following method. The activity of lipase was determined by measuring the rate of increase in absorbance due to release of p-nitrophenol by the action of lipase. A mixture obtained by adding p-nitrophenylbutyric acid (pNPB) (SIGMA) at a final concentration of 2 mM to 20 mM Tris-HCl (pH 7.0), and mixing them, was used as a substrate solution. 4 µL of the culture supernatant diluted with 20 mM Tris-HCl (pH 7.0) as appropriate and 100 µL of the substrate solution were mixed in each well of a 96-well assay plate, and an absorbance change (OD/min) at 405 nm was measured at 30°C. A difference ΔOD/min in the absorbance change from a blank (a culture supernatant-free sample) was determined. The lipase activity of recombinant *Bacillus* bacteria with an expression cassette containing the modified-5'UTR was given as a relative value (a.u.) when the lipase activity of recombinant *Bacillus* bacteria with an expression cassette containing the unmodified 5'-UTR is assumed to be 1.

### 2) Results

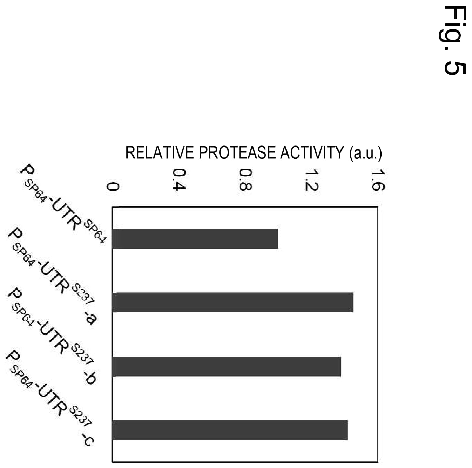

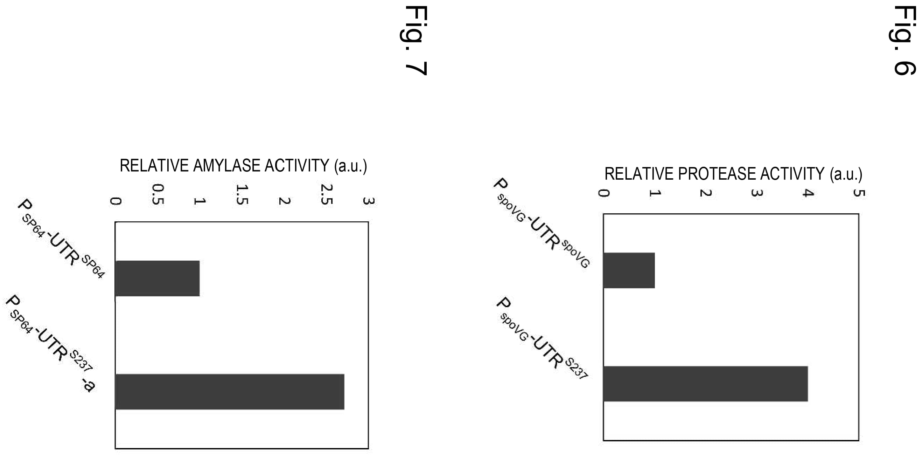

Fig. 5 shows the improvement of the amount of KP43 protease produced by recombinant *Bacillus* bacteria with the KP43 protease expression cassette having the modified P_{SP64} promoter (P_{SP64}-UTR^{S237}-a, b and c) containing the modified 5'-UTR, which was constructed in Example 2. When the amount of KP43 protease produced by use of a parent promoter (P_{SP64}-UTR^{SP64}) containing the unmodified 5'-UTR is assumed to be 100%, P_{SP64}-UTR^{S237}-a, b and c containing the modified 5'-UTR improved the amount of protease produced in recombinant *Bacillus* bacteria by 45%, 38% and 42%, respectively, as compared to the parent promoter. Fig. 6 shows the improvement of the amount of KP43 protease produced by recombinant *Bacillus* bacteria with the KP43 protease expression cassette having the spoVG promoter (P_{spoVG}-UTR^{S237}) containing the modified 5'-UTR, which was constructed in Example 3. P_{spoVG}-UTR^{S237} containing the modified 5'-UTR improved the amount of protease produced in recombinant *Bacillus* bacteria by a factor of 4.0 as compared to the parent promoter (P_{spoVG}-UTR^{spoVG}) containing the unmodified 5'-UTR.

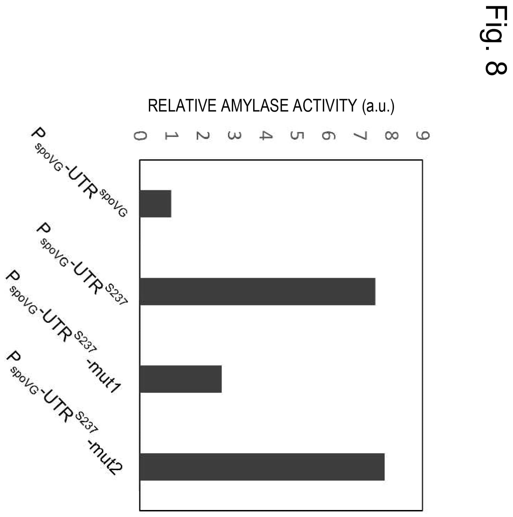

Fig. 7 shows the improvement of the amount of amylase produced by recombinant *Bacillus* bacteria with the amylase expression cassette having the modified P_{SP64} promoter (P_{SP64}-UTR^{S237}-a) containing the modified 5'-UTR, which was constructed in Example 4. P_{SP64}-UTR^{S237}-ₐ containing modified 5'-UTR improved the amount of amylase produced in recombinant *Bacillus* bacteria by a factor of 2.7 as compared to the parent promoter (P_{SP64}-UTR^{SP64}) containing the unmodified 5'-UTR. Fig. 8 shows the improvement of the amount of amylase produced in recombinant *Bacillus* bacteria with the amylase expression cassette having the spoVG promoter (P_{spoVG}-UTR^{S237}, P_{spoVG}-UTR^{S237}-mut1 and P_{spoVG}-UTR^{S237}-mut2) containing the modified 5'-UTR, which was constructed in Example 4. P_{spoVG}-UTR^{S237}, P_{spoVG}-UTR^{S237}-mut1 and P_{spoVG}-UTR^{S237}-mut2 containing the modified 5'-UTR improved the amount of amylase produced in recombinant *Bacillus* bacteria by factors of 7.5, 2.6 and 7.8, respectively, as compared to the parent promoter (P_{spoVG}-UTR^{spoVG}) containing the unmodified 5'-UTR.

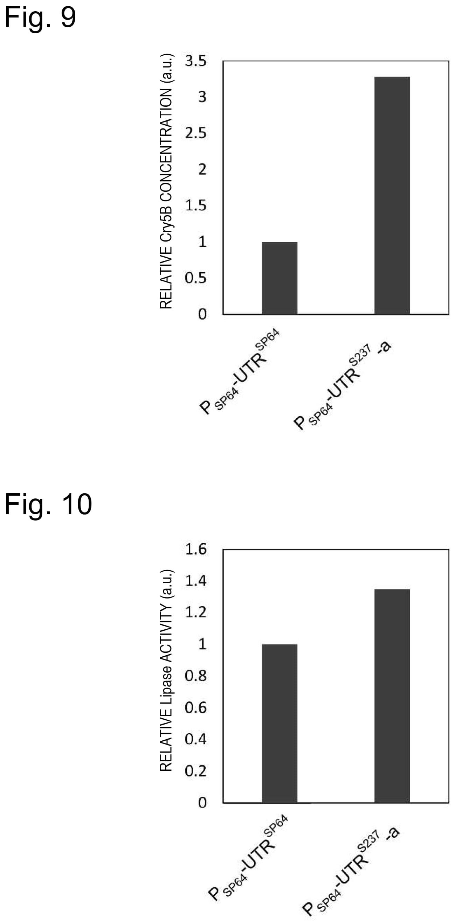

Fig. 9 shows the improvement of the amount of Cry5B produced by recombinant *Bacillus* bacteria with the Cry5B expression cassette having the modified P_{SP64} promoter (P_{SP64}-UTR^{S237}-a) containing the modified 5'-UTR, which was constructed in Example 5. P_{SP64}-UTR^{S237}-a containing the modified 5'-UTR improved the amount of Cry5B produced in recombinant *Bacillus* bacteria by a factor of 3.3 as compared to the parent promoter (P_{SP64}-UTR^{SP64}) containing the unmodified 5'-UTR.

Fig. 10 shows the improvement of the amount of lipase produced by recombinant *Bacillus* bacteria with the lipase expression cassette having the modified P_{SP64} promoter (P_{SP64}-UTR^{S237}-a) containing the modified 5'-UTR, which was constructed in Example 6. P_{SP64}-UTR^{S237}-ₐ containing the modified 5'-UTR improved the amount of lipase produced in recombinant *Bacillus* bacteria by 34% as compared to the parent promoter (P_{SP64}-UTR^{SP64}) containing the unmodified 5'-UTR.

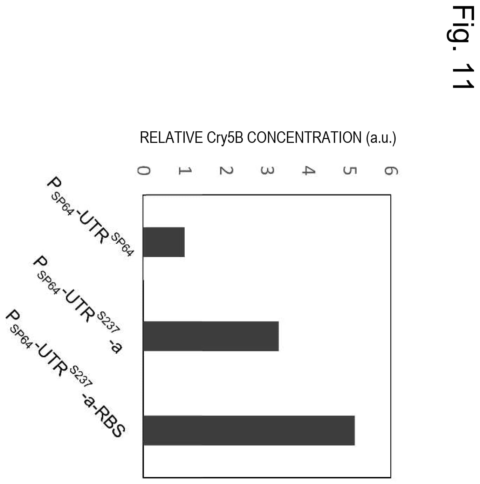

Fig. 11 shows the improvement of the amount of Cry5B produced by recombinant *Bacillus* bacteria with the Cry5B expression cassette having the modified P_{SP64} promoter (P_{SP64}-UTR_{S237}-a-RBS) containing the modified 5'-UTR having additional RBS, which was constructed in Example 7. When the amount of Cry5B produced by use of the modified promoter (P_{SP64}-UTR^{S237}-a) having no additional RBS is assumed to be 100%, the modified promoter (P_{SP64}-UTR^{S237}-a-RBS) having additional RBS improved the amount of Cry5B produced by 56% as compared to P_{SP64}-UTR^{S237}-a. P_{SP64}-UTR_{S237}-a-RBS improved the amount of Cry5B produced in recombinant *Bacillus* bacteria by a factor of 5.1 as compared to the parent promoter (P_{SP64}-UTR^{SP64}) containing the unmodified 5'-UTR.

## Claims

1. A DNA molecule comprising a modified promoter,
the modified promoter comprising a modified 5'-UTR containing a heterologous polynucleotide,
the heterologous polynucleotide comprising a nucleotide sequence of SEQ ID NO: 70, or a nucleotide sequence having at least 80% identity with the nucleotide sequence of SEQ ID NO: 70.

2. The DNA molecule according to claim 1, wherein the heterologous polynucleotide is any of the following polynucleotides a) to d):
a) a polynucleotide comprising a nucleotide sequence of SEQ ID NO: 3, or a nucleotide sequence having at least 80% identity with the nucleotide sequence of SEQ ID NO: 3;
b) a polynucleotide consisting of a nucleotide sequence of SEQ ID NO: 4, or a nucleotide sequence having at least 80% identity with the nucleotide sequence of SEQ ID NO: 4, or a fragment thereof;
c) a polynucleotide comprising a nucleotide sequence of SEQ ID NO: 5, or a nucleotide sequence having at least 80% identity with the nucleotide sequence of SEQ ID NO: 5; and
d) a polynucleotide comprising a nucleotide sequence of SEQ ID NO: 6, or a nucleotide sequence having at least 80% identity with the nucleotide sequence of SEQ ID NO: 6.

3. The DNA molecule according to claim 1 or 2, wherein the heterologous polynucleotide comprises a nucleotide sequence of SEQ ID NO: 1 or SEQ ID NO: 2.

4. The DNA molecule according to any one of claims 1 to 3, wherein the heterologous polynucleotide has a length of 100 nt or less.

5. The DNA molecule according to any one of claims 1 to 4, wherein the heterologous polynucleotide is located at a 3'-end of the modified 5'-UTR.

6. The DNA molecule according to any one of claims 1 to 5, wherein the modified 5'-UTR comprises a heterologous ribosome binding sequence in addition to the heterologous polynucleotide, and the ribosome binding sequence is arranged upstream of a region consisting of the nucleotide sequence of SEQ ID NO: 1 contained in the modified 5'-UTR.

7. The DNA molecule according to claim 6, wherein the heterologous ribosome binding sequence is arranged upstream of the heterologous polynucleotide.

8. The DNA molecule according to claim 6 or 7, wherein the heterologous ribosome binding sequence is located 10 to 100 nucleotides downstream of a transcription start site of the modified promoter.

9. The DNA molecule according to any one of claims 1 to 8, wherein a parent promoter of the modified promoter is derived from *Bacillus* bacteria.

10. The DNA molecule according to claim 9, wherein the parent promoter is a polynucleotide consisting of a nucleotide sequence of SEQ ID NO: 72, or a nucleotide sequence having at least 80% identity with the sequence of SEQ ID NO: 72, or a polynucleotide consisting of a nucleotide sequence of SEQ ID NO: 76, or a nucleotide sequence having at least 80% identity with the sequence of SEQ ID NO: 76.

11. The DNA molecule according to any one of claims 1 to 10, further comprising a target gene.

12. The DNA molecule according to claim 11, which is an expression cassette or an expression vector.

13. A transformant comprising the DNA molecule according to claim 12.

14. The transformant according to claim 13, wherein the transformant comprises the DNA molecule according to claim 12, wherein the DNA molecule is an expression cassette which contains the modified promoter and further contains a target gene, and
when an expression level of the target gene in cells comprising an expression cassette which contains a parent promoter of the modified promoter and further contains the target gene is assumed to be 100%,
the transformant increases the expression level of the target gene by at least 10%.

15. The transformant according to claim 13 or 14, which is *Bacillus* bacteria.

16. A method for producing a target substance, comprising culturing the transformant according to any one of claims 13 to 15.

17. A method for producing a modified promoter,
the method comprising modifying a 5'-UTR contained in a parent promoter,
wherein the modifying a 5'-UTR comprises substituting a part or a whole of the 5'-UTR with a heterologous polynucleotide, or adding the heterologous polynucleotide to the 5'-UTR,
the heterologous polynucleotide comprising a nucleotide sequence of SEQ ID NO: 70, or a nucleotide sequence having at least 80% identity with the nucleotide sequence of SEQ ID NO: 70,
the parent promoter comprising a 5'-UTR which does not contain the heterologous polynucleotide.
